# EUROPEAN PATENT APPLICATION

(11) **EP 1 555 029 A1**
(43) Date of publication of application: **20.07.2005**
(21) Application number: 04000975.5
(22) Date of filing: 19.01.2004
(51) Int. Cl.: A61K 38/11, A61K 38/08, A61P 15/06

(54) **Use of substances having oxytocin antagonistic properties for the preparation of a medicament for treating hypertension**

(71) Applicant: Ferring B.V., 2132 JX Hoofddorp (NL)
(72) Inventor: Reinheimer, Torsten, Michael, DK-1809 Frederiksberg C (DK); Garfield, Robert Edward, Friendswood Texas 77546 (US)
(74) Representative: Poulsen, Niels Jakob

(57) **Abstract**

The present invention relates to uses of a substance having oxytocin antagonistic properties and to methods of treatment comprising administration of a pharmaceutically effective amount of said substance.

## Description

### Technical field of the invention

The present invention relates to uses of a substance having oxytocin antagonistic properties and to methods of treatment comprising administration of a pharmaceutically effective amount of said substance.

### Background

It is known that oxytocin is a cardiovascular hormone and paracrine transmitter and oxytocin or oxytocin receptors are expressed in e.g. heart, vascular endothelium and smooth muscle, and kidney (epithelium).

Hypertension is a common disorder worldwide in which blood pressure remains abnormally high (a value of 140/90 mm Hg or more) due to the fact that arterioles throughout the body stay constricted, driving up the pressure in the larger blood vessels. Thereby, the blood flows through the vessels at a greater than normal pressure. High blood pressure strains the heart; harms the arteries; and increases the risk of heart attack, stroke, and kidney diseases.

Preeclampsia is a cardiovascular disorder that occurs only during pregnancy and affects both the mother and the unborn foetus. Affecting at least 5 percent of all pregnancies, it is a rapidly progressive condition characterized by high blood pressure, swelling (edema), protein excretion in the urine and fetal growth restriction. Sudden weight gain, headaches and changes in vision are important symptoms; however, some women with rapidly advancing disease report only few symptoms. Typically, preeclampsia occurs in the late 2nd or 3rd trimesters (middle to late pregnancy), though symptoms may arise earlier. Further, children of mothers having preeclampsia run a higher risk of being subjected to inhibition of growth. Proper prenatal care is essential to diagnose and manage preeclampsia. Preeclampsia, pregnancy induced hypertension (PIH) and toxemia are closely related conditions. The HELLP syndrome and eclampsia are more severe forms.

The HELLP syndrome, is a unique variant of preeclampsia (toxemia), and stands for: H (hemolysis, which is the breaking down of red blood cells), EL (elevated liver enzymes), and LP (low platelet count). It can be fatal to both the mother and the baby. The HELLP syndrome occurs in tandem with preeclampsia. In view of the fact that HELLP Syndrome's symptoms may happen before preeclampsia's three findings (high blood pressure, protein excretion in the urine, and swelling (edema)), they may be misdiagnosed as symptoms of gastritis, disseminated intravascular coagulation (DIC), acute hepatitis, gall bladder disease, and other conditions. As a result, the mother may not get the right treatment, leaving both mother and baby at a higher risk.

Preeclampsia and other hypertensive disorders of pregnancy are a leading global cause of maternal and infant illness and death. By conservative estimates, these disorders are responsible for approximately 50,000 to 76,000 deaths worldwide each year. The only known effective treatment is delivery of the foetus. Symptomatic treatment and treatment of severe preeclampsia is usually achieved with MgSO₄ or other antihypertensive agents.

Intrauterine growth retardation (IUGR), which is defined as less than 10 percentile of predicted fetal weight for gestational age, may result in significant fetal morbidity and mortality if not properly diagnosed. The condition is most commonly caused by inadequate maternal-fetal circulation, with a resultant decrease or inhibition in fetal growth and development. Less common causes include intrauterine infections such as cytomegalovirus and rubella, and congenital anomalies such as trisomy 21 and trisomy 18. When IUGR is recognized, it is important to attempt to correct reversible causes, although many of the conditions responsible for IUGR are not amenable to antenatal therapy. Close fetal surveillance with delivery before 38 weeks of gestation is usually recommended. Some infants born with IUGR have cognitive and medical problems, such as problems in the development of organs and infection of the foetus before birth, i.e. congenital infections, although for most infants the long-term prognosis is good. IUGR is common in preeclampsia.

In US 5,962,413 dysmenorrhea, disfunctional uterine bleeding, preterm labor and postpartum labor in female mammals are treated by inhibiting uterine contractility by administering thereto a nitric oxide synthase substrate, a nitric oxide donor or both, in combination with one or more of a prostaglandin inhibitor, a prostacyclinmimetic, a progestin, an oxytocin antagonist or a beta-agonist in an amount effective to ameliorate the symptoms thereof; and inadequate menses and induction of abortion or stimulation of labor in a pregnant female is achieved by uterine contractility stimulation by administering thereto a nitric oxide inhibitor, either alone or in combination with progesterone antagonist, an oxytocin or oxytocin analogue or a prostaglandin. Thus, the object of US 5,962,413 is a method of regulating the nitric oxide dependent contractility of the uterine muscle of a female mammal.

The present invention differs from US5,962,413 in that it does not rely on treatment of contraction disorders with compounds influencing the NO pathways.

In view of the lack of treatments of the diseases mentioned above, there is thus a need to provide treatment of hypertension and diseases where hypertension is involved, especially as there are no specific/causal treatments available today for certain of said diseases.

### Summary of the invention

The present invention relates in one aspect to the use of a substance having oxytocin antagonistic properties for the manufacture of a medicament for the treatment of a disease relating to oxytocin-induced vascular contractility.

The present invention relates in another aspect to a method of treatment comprising administration of a pharmaceutically effective amount of a substance having oxytocin antagonistic properties to a subject suffering from a disease relating to oxytocin-induced vascular contractility.

### Brief description of the figures

Fig. 1 displays the effect of oxytocin antagonists on blood pressure in pregnant rats. The X axis represents the time of pregnancy (gestational age), and the Y axis represents the systolic blood pressure (mmHg). Barusiban (200 µg/d), atosiban (200 µg/d) and control (saline) (200 µg/d) were administered to pregnant rats from gestational day 19 to 22 and during pp1 to pp7 (pp=post partum). Especially, for barusiban there is a beneficial drop in systolic blood pressure around birth.

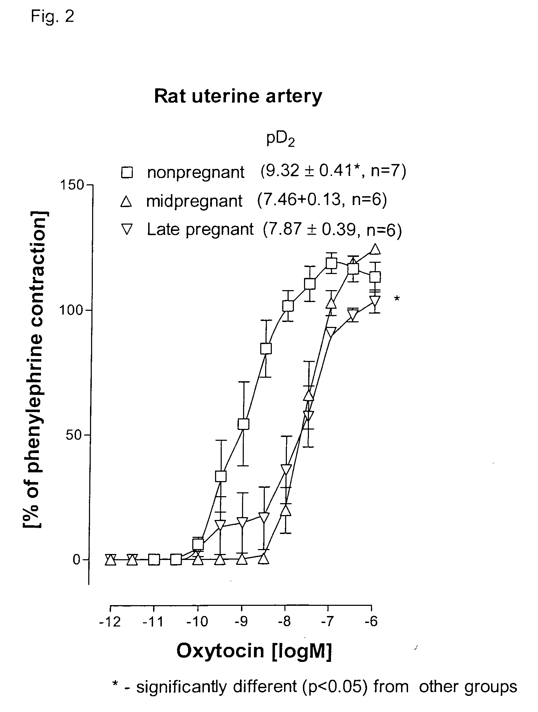

Fig. 2 displays administration of oxytocin to isolated rat uterine artery in vitro and effects on contractility from nonpregnant, midpregnant and late pregnant rats in order to compare oxytocin sensitivity. The X axis represents the oxytocin concentration [log M] and the Y axis represents % of phenylephrine-induced contraction. Uterine arteries of non-pregnant rats showed a higher sensitivity to oxytocin compared to mid or late pregnant animals. The following pD₂ values were obtained; non-pregnant (9.32 ± 0.41, n=7), midpregnant (7.46 ± 0.13, n=6) and late pregnant (7.87 ± 0.39, n=6). It could be speculated that the generally lower sensitivity of uterine vessels during pregnancy is related to eventually higher local oxytocin concentrations.

Fig. 3 displays contraction dose/response relationships and oxytocin sensitivity of isolated uterine artery, femoral artery, carotoid artery and aorta from non-pregnant spontaneous hypertensive (SHR) rats. The uterine arteries show the highest sensitivity.

Fig. 4 displays contraction dose/response relationships and oxytocin sensitivity of isolated uterine artery, femoral artery, carotoid artery and aorta from non-pregnant Wistar (WKY) rats. The uterine arteries have an outstanding high sensitivity compared to the others.

Fig. 5 displays contraction dose/response relationships and oxytocin sensitivity between uterine arteries from non-pregnant WKY and SHR rats. The Wistar (WKY) rats show a higher sensitivity.

The uterine vessels have an exceptional high oxytocin sensitivity compared to other vessels demonstrating the critical role of the endogenous and (exogenous oxytocin) tone for the vascular tone of the uterine vessels. The contraction or dilatation of these vessels has direct impact on the perfusion of the foetus, placenta and uterus. Contraction of uterine blood vessels and hypoperfusion of the foetus is directly associated with diseases such as IUGR or preeclampsia. The new use of oxytocin antagonists according to the invention promises direct uterine vasodilatation and improvement of disease related symptoms for mother and foetus and a better neonatal outcome.

Thus, the presented experiments in the figures have demonstrated proof of the concept of the present invention in rats, in vivo as well as in isolated arteries.

### Detailed description of the invention

In an embodiment of the use of the invention said substance is a peptide.

In a further embodiment of the use of the invention said peptide is chosen from the group comprising pentapeptides, hexapeptides, heptapeptides, octapeptides, nonapeptides and decapeptides.

In a another embodiment of the use of the invention said peptide is chosen from the group comprising atosiban, ([Mpa1, D-Tyr(Et)2, Thr4, Orn8]-oxytocin, barusiban, C^{4,6}, S¹-cyclo[N-(3-sulfanylpropanoyl)-D-tryptophyl-L-isoleucyl-L-alloisoleucyl-L-asparaginyl-L-2-aminobutanoyl-N-methyl-L-ornithinol] and pharmaceutically acceptable salts thereof.

In an embodiment of the use of the invention said substance having antagonistic properties is a heptapeptide analogue, or a pharmaceutically acceptable salt thereof, having oxytocin antagonist activity and consisting of a hexapeptide moiety S and a C-terminal β-aminoalcohol residue Z bound to the moiety S by an amide bond, wherein the β-aminoalcohol is: 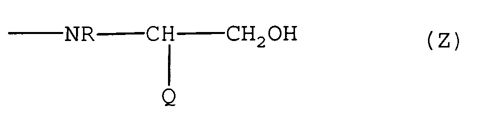 wherein Q is (CH₂)ₙ-NH-A, n is 1-6 and A is H or
-C(=NH)NH₂, and wherein R is CH₃ or C₂H₅; and the moiety S is: 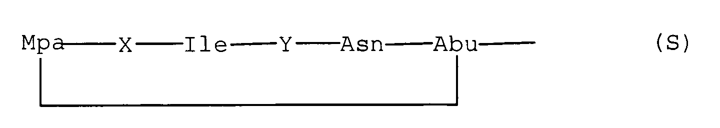 wherein Mpa, Ile, Asn and Abu have the following meaning:
- Mpa: 3-mercaptopropionic acid residue
- Ile: isoleucine residue
- Asn: asparagine residue
- Abu: α-aminobutyric acid residue;
and wherein
- X: is a D-aromatic α-amino acid; and
- Y: is an aliphatic acid residue.

The heptapeptide analogue above is disclosed in EP 0 938 496 B1.

In a further embodiment of the use of the invention said substance having antagonistic properties is a vasotocin derivative, characterized in that it has the formula 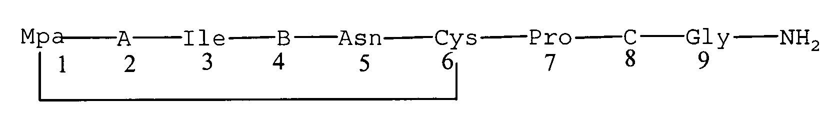 wherein
Mpa is a 3-mercaptopropionoyl residue(-S-CH₂-CH₂-CO-) ;
A is the peptide residue of L- or D-tyrosine-O-ethyl ether (L- or D-Tyr(Et)), or of a hydrophobic D-amino acid;
Ile is the peptide residue of isoleucine;
B is the peptide residue of glutamine (Gln), threonine (Thr) or valine (Val);
Asn is the peptide residue of asparagine;
Cys is the peptide residue of cysteine;
Pro is the peptide residue of proline;
C is the peptide residue of L- or D-arginine (Arg), ornithine (Orn) or citrulline (Cit); and
Gly-NH₂ is the peptide residue of glycine amide.

Above vasotocin derivatives are disclosed in EP 0 112 809 B1.

In a further embodiment of the use of the invention said medicament is for the treatment of hypertension and for the treatment of hypertension relating to pregnancy.

In a yet further embodiment of the invention said oxytocin-induced vascular contractility is present in uterine vessels, vessels in placenta and foetus.

In a further embodiment of the use of the invention said oxytocin-induced vascular contractility is present in vessels in the heart, the kidney, the brain, the thymus, the pancreas or any other vessels. The effect of the use of oxytocin antagonists according to the invention is expected to be seen in any vessels of the body of a mammal, preferably a human, where oxytocin receptors are present.

In another embodiment of the use of the invention said medicament is for the treatment of preeclampsia, intrauterine growth retardation (IUGR), hypoperfusion of the foetus, pregnancy induced hypertension (PIH), toxemia, HELLP syndrome or eclampsia.

As for the method of the invention, said substance is preferably a peptide.

In a further embodiment of the method of the invention said peptide is chosen from the group comprising pentapeptides, hexapeptides, heptapeptides, octapeptides, nonapeptides and decapeptides.

In a further embodiment of the method of the invention said peptide is chosen from the group comprising atosiban, barusiban and pharmaceutically acceptable salts thereof.

In a further embodiment of the method of the invention said medicament is for the treatment of hypertension or for the treatment of hypertension relating to pregnancy.

In another embodiment of the method of the invention said oxytocin-induced vascular contractility is present in uterine vessels, vessels in placenta or foetus.

In a further embodiment of the method of the invention said oxytocin-induced vascular contractility is present in vessels in the heart, the kidney, the brain, the thymus, the pancreas or any other vessels.

In a further embodiment of the method of the invention said medicament is for the treatment of preeclampsia, intrauterine growth retardation (IUGR), hypoperfusion of the foetus, pregnancy induced hypertension (PIH), toxemia, HELLP syndrome or eclampsia.

In an embodiment of the invention said subject to be treated with the oxytocin antagonist is a mammal, preferably a human.

By the term "pharmaceutically effective amount of a substance", as used herein, is meant to include an amount of the substance in the range from about 0,05 mg to about 500 mg, preferably from about 1 to about 350 mg, in a single or multiple dose, per patient and day. The dose should be high enough to achieve the relaxing effects on the oxytocin-induced vascular contractility of the vessels in the body without any negative side effects.

By the term "substance" as used herein is meant to comprise small molecules, peptides, non-peptides, peptide-mimetics or any other molecules having oxytocin antagonistic properties.

By the term "pentapeptide" as used herein is meant a compound comprising at least 5 peptide bonds. The definition is applied analogously to hexapeptides, heptapeptides, octapeptides, nonapeptides and decapeptides.

The oxytocin antagonist may be present in a solution for injection suitable for administration to a patient. The solution for injection comprises, in addition to the oxytocin antagonist, any excipient, e.g. mannitol, hydrochloric acid 1 M and water for injection, conventionally used within the art of solutions for injections. The oxytocin antagonist may also be present in a concentrate for solution for infusion. However, the oxytocin antagonist may also be present in any other formulation such as a nasal spray suitable for administration to a patient.

The substance, e.g. atosiban or barusiban, may be administered in a 7.5 mg/ml solution for injection to a patient, i.e. 1 ml solution contains 7.5 mg substance free base in the form of e.g. atosiban or barusiban acetate. The administration may be started with a bolus injection and followed by infusion, e.g. a 0.9 ml intravenous bolus injection over approximately 1 minute per patient and therapy regimen (dose of 6,75 mg atosiban), thereafter 3 hours intravenous loading infusion of 24 ml/hour per patient and regimen (total dose atosiban of 18 mg/hour), and subsequent intravenous infusion of 8 ml/hour per patient and regimen (total dose of atosiban 6 mg/hour). The doses above may be adjusted in order to suit the particular medical situation.

The invention describes a new use of oxytocin antagonists for the treatment of hypertension and diseases where hypertension is involved. Data provided herein show that oxytocin can regulate vascular contractility and stimulate contractions or increase blood pressure. Both vascular contractility and blood pressure are inhibited by oxytocin antagonists according to the studies performed herein.

Therefore, these studies imply that the oxytocin antagonists can be used to lower high blood pressure and that they might be used therapeutically in cases of hypertension of individuals suffering from such conditions and hypertension related conditions found in pregnant women, particularly hypertension associated with preeclampsia and IUGR.

Additionally, according to the present invention a use of oxytocin antagonists improving the perfusion between mother and foetus by relaxing relevant blood vessels (e.g. uterine vessels, vessels in the placenta and foetus) is provided. Direct dilatation of uterine arteries improves perfusion of foetus. The new use of the oxytocin antagonists according to the invention would inevitably improve perfusion, nutrition, growth and development of the foetus and a better health for both mother and foetus is expected.

In the case of e.g. preeclampsia where there are no effective treatments available today, but delivering the child, according to the present invention an effective causal treatment is provided with the new use of oxytocin antagonists according to the invention.

It is further expected that a high selectivity, high potency and efficacy of the oxytocin antagonist barusiban and variants of barusiban will be obtained. No substantial side effects are neither expected from the new use of the oxytocin antagonist according to the invention.

### Examples

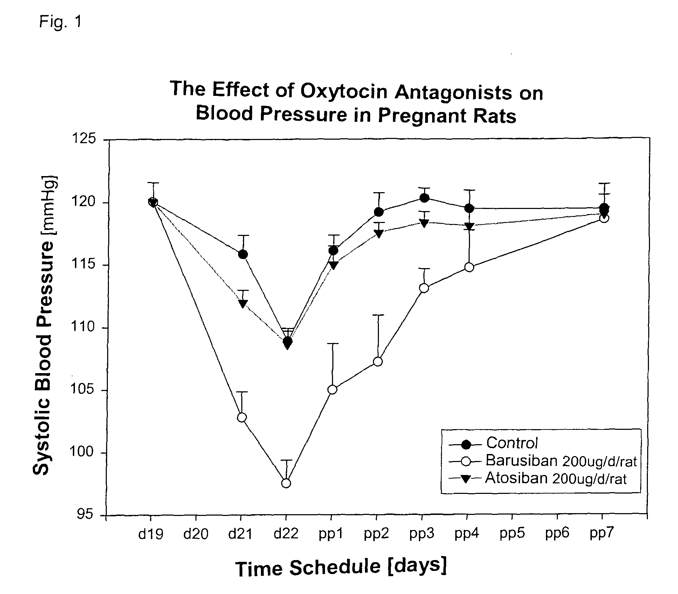

### Blood pressure measurements in rats (figure 1)

Animals. Adult nulliparous pregnant rats (300-325 g body weight) were purchased from Harlan Sprague Dawley, Inc (Indianapolis, Ind), and were received in the animal facilities on day 16 of pregnancy (with day 1 being the day of the positive sperm smear result). All animals were given free access to food and water.

Blood pressure measurement. Starting on day 19 of pregnancy systolic blood pressure was measured daily or every other day with a pneumatic tail cuff device (NARCO Biosystems, Inc, Austin, Tex) in animals that had been warmed in a metal chamber maintained at approximately 30°C. Blood pressure values obtained from 3 consecutive measurements were averaged and recorded as the pressure for a given rat at each point. Measurements of systolic blood pressure of all animals were carried out until delivery and for seven days post partum (pp7). All animals delivered on day 22 of gestation, the expected day of delivery. Three groups of animals were studied: Group 1. Control group treated with saline, Group 2. Barusiban - treated (200 ug/day), and Group 3. Atosiban-treated (200 ug/day).

Statistical analysis. Mean +/- SEM values were calculated for blood pressure levels on each day of pregnancy and post partum. Statistical analysis was performed with 1-way analysis of variance followed by multiple comparison with the Fisher least significant difference criteria. P < .05 was considered significant.

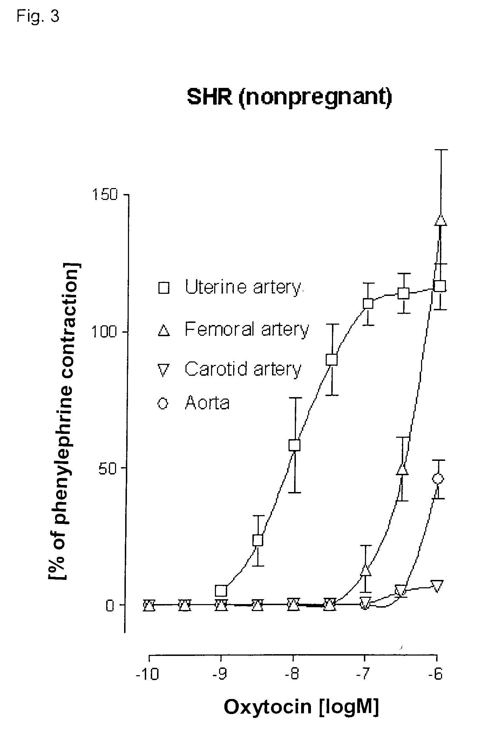

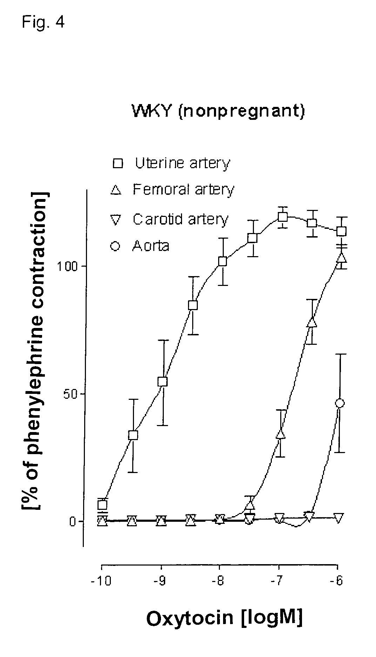

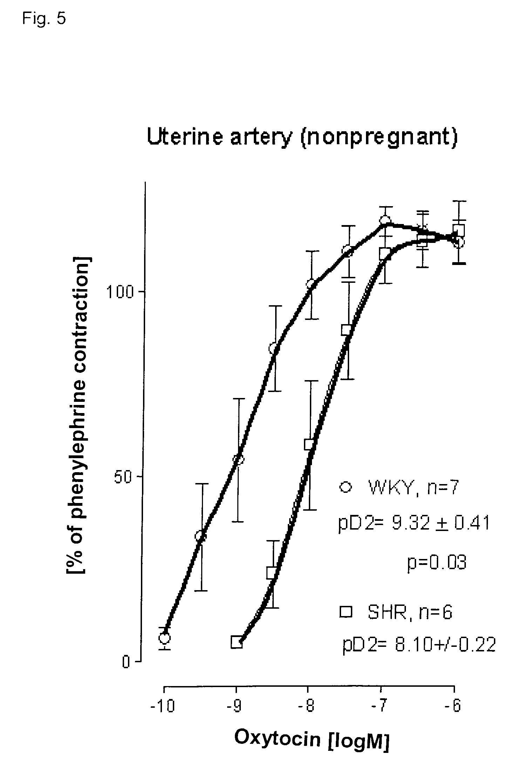

### In Vitro contractility methods (figures 2-5):

Nonpregnant or timed-pregnant Sprague-Dawley rats (of a 22-day pregnancy, with day 1 being the day when the sperm plug was observed) were obtained from Charles River Laboratories (Houston, Texas). In some studies Spontaneously Hypertensive Rats (SHR) and their respective normotensive controls (WKY) were used. Rats were killed by means of carbon dioxide inhalation on day 14 (midterm pregnant) or day 22 before labor (term pregnant) or during the estrus cycle (nonpregnant). The vascular tissues (uterine artery, femoral artery, carotid artery, or aorta) were excised through a midline abdominal incision and placed in cold Krebs' buffer of the following composition: sodium chloride, 119.0 mmol/l; potassium chloride, 4.7 mmol/l; potassium phosphate, 1.2 mmol/l; sodium bicarbonate, 25 mmol/l; magnesium sulfate, 1.2 mmol/l, calcium chloride, 2.5 mmol/l, sodium ethylenediaminetetraacetic acid, 0.026 mmol/l, and glucose, 11.5 mmol/l. Rings 5 mm in width were cut from the middle of the vascular segment and placed in organ chambers between 2 tungsten stirrups for tension recording with an isometric force transducer (Harvard Apparatus, South Natick, Mass). Rings were equilibrated in organ chambers containing 10 ml of Krebs' buffer maintained at 37°C and continuously bubbled with 5% carbon dioxide in air (approximately pH 7.40) at 2 g of passive tension until stabilized. Contractions of uterine vascular tissues (cut into rings) were recorded and stored on computer with the DI-220 (DataQ Instruments Inc, Akron, Ohio) data acquisition system. The oxytocin was added directly to the organ chambers in 1.0-log unit increments in volumes of 10 *µ*l (10⁻⁹-10⁻⁵ mol/l) at 10-minute intervals. After 15 minutes of exposure to the last concentration of oxytocin the organ chambers and tissues were then washed of the oxytocin and respective antagonists, and after 30 minutes of rest, 60-mmol/l potassium chloride or phenylephrine was applied to check the viability of the tissues and maximum induced contractions to these vasokonstrictors.

Data were analyzed by using Windaq/200 (DataQ Instruments Inc) software. Integral activity as an area under the tracing of vascular contractions for 10 minutes (expressed as milligrams per second) before (basal activity) and after the addition of each concentration of the agent was calculated and compared with basal activity (as percentage change). A time-solvent control (solvent is deionized water) was run in parallel in these experiments, and the changes were subtracted. The concentration of oxytocin causing 50% of the maximal effect was determined, and the -log₅₀ of the half maximal effect and the area under the concentration-response curves were calculated and compared. The data are expressed as mean ± SD. One-way analysis of variance and Tukey multiple comparison tests were used as appropriate. A 2-tailed P value <0.05 was considered statistically significant.

## Claims

1. Use of a substance having oxytocin antagonistic properties for the manufacture of a medicament for the treatment of a disease relating to oxytocin-induced vascular contractility.

2. Use of a substance according to claim 1, wherein said substance is a peptide.

3. Use according to claim 2, wherein said peptide is chosen from the group comprising pentapeptides, hexapeptides, heptapeptides, octapeptides, nonapeptides and decapeptides.

4. Use according to claim 3, wherein said peptide is chosen from the group comprising atosiban, barusiban and pharmaceutically acceptable salts thereof.

5. Use according to any one of claims 1-4, wherein said medicament is for the treatment of hypertension.

6. Use according to claim 5, wherein said medicament is for the treatment of hypertension relating to pregnancy.

7. Use according to any one of claims 1-6, wherein said oxytocin-induced vascular contractility is present in uterine vessels, vessels in placenta or foetus.

8. Use according to any one of claims 1-5, wherein said oxytocin-induced vascular contractility is present in vessels in the heart, the kidneys, the brain, the thymus, the pancreas or any other vessels.

9. Use according to any one of claims 1-7, wherein said medicament is for the treatment of preeclampsia, intrauterine growth retardation (IUGR), hypoperfusion of the foetus, pregnancy induced hypertension (PIH), toxemia, HELLP syndrome or eclampsia.

10. A method of treatment comprising administration of a pharmaceutically effective amount of a substance having oxytocin antagonistic properties to a subject suffering from a disease relating to oxytocin-induced vascular contractility.

11. A method according to claim 10, wherein said substance is a peptide.

12. A method according to claim 11, wherein said peptide is chosen from the group comprising pentapeptides, hexapeptides, heptapeptides, octapeptides, nonapeptides and decapeptides.

13. A method according to claim 12, wherein said peptide is chosen from the group comprising atosiban, barusiban and pharmaceutically acceptable salts thereof.

14. A method according to any one of claims 10-13, wherein said disease is hypertension.

15. A method according to claim 14, wherein said hypertension relates to pregnancy.

16. A method according to any one of claims 10-15, wherein said oxytocin-induced vascular contractility is present in uterine vessels, vessels in placenta or foetus.

17. A method according to any one of claims 10-14, wherein said oxytocin-induced vascular contractility is present in the heart, the kidney, the brain, the thymus, the pancreas or any other vessels.

18. A method according to any one of claims 10-16, wherein said disease is preeclampsia, intrauterine growth retardation (IUGR), hypoperfusion of the foetus, pregnancy induced hypertension (PIH), toxemia, HELLP syndrome or eclampsia.

19. A method according to any one of claims 10-18, wherein said subject is a human.
